# EUROPEAN PATENT APPLICATION

(11) **EP 3 777 819 A1**
(43) Date of publication of application: **17.02.2021**
(21) Application number: 19777809.5
(22) Date of filing: 28.03.2019
(51) Int. Cl.: A61K 8/29, A61K 8/02, A61K 8/24, A61Q 1/00

(54) **WHITE PIGMENT FOR COSMETICS, AND COSMETIC**

(30) Priority: 30.03.2018 JP 2018068328; 30.03.2018 JP 2018068334
(71) Applicant: Fujimi Incorporated, Kiyosu-shi, Aichi 452-8502 (JP)
(72) Inventor: IWAKUNI Mayumi, Kiyosu-shi, Aichi 452-8502 (JP); ASHITAKA Keiji, Kiyosu-shi, Aichi 452-8502 (JP); MIWA Naoya, Kiyosu-shi, Aichi 452-8502 (JP)
(74) Representative: Grünecker Patent- und Rechtsanwälte PartG mbB
(86) International application number: PCT/JP2019/013798
(87) International publication number: WO 2019/189665

(57) **Abstract**

Provided is a white pigment for cosmetics, and the white pigment has a higher function as a base pigment than that of titanium oxide. A white pigment for cosmetics of the present invention includes a titanium phosphate powder having a whiteness of 92.91 or more as determined in accordance with JIS Z 8715.

## Description

### Technical Field

The present invention relates to a white pigment for cosmetics.

### Background Art

White pigments for cosmetics are a base pigment that is mixed with other pigments to give a composition of a cosmetic, and a white pigment having a higher whiteness can exhibit a higher function as the base pigment. As a conventional white pigment for cosmetics, titanium(IV) oxide (TiO₂, titanium dioxide, hereinafter also simply called "titanium oxide") is typically used.

As for a cosmetic containing titanium oxide as a white pigment, for example, PTL 1 discloses a powder cosmetic including a titanium dioxide powder satisfying the following constitution (a) and an iron-containing powder satisfying the following constitution (b).
(a) : The titanium dioxide powder is a powder having an average particle diameter of 1.5 to 2.5 µm and a whiteness of 97.0 or more, and the powder is pressed to give a molded product having a disintegration strength of 140 g or less. The whiteness is determined as follows: 10 g of titanium dioxide is packed in a round aluminum plate having a diameter of 5.2 cm and a thickness of 2 mm and is pressed at 67.5 kg/cm² with a hydraulic stamping press machine manufactured by Riken Seiki; and the resulting molded product is subjected to measurement with a spectroscopic colorimeter, SE-2000, manufactured by Nippon Denshoku Industries Co., Ltd. to give a lightness value (L value) as the whiteness.
(b) : The iron-containing powder has a color tone of, in terms of Munsell color values, hues of 0.00R to 10.00R, 0.00YR to 10.00YR, and 0.00Y to 10.00Y, a lightness of 3.00 to 9.00, and a color saturation of 1.00 to 12.00.

The document also discloses, as effects achieved by the powder cosmetic, that the powder cosmetic gives a small change in color between an appearance color and an application color, gives no white powdery finish, and gives excellent smoothness on the skin and natural skin color finish.

PTL 2 discloses a cosmetic including titanium oxide as the material of a white pigment. The cosmetic includes white pigment spherical microparticles, and the white pigment spherical microparticles specifically have an average particle diameter of 80 to 800 nm and a coefficient of particle diameter variation of 10% or less. The white pigment spherical microparticles are, for example, spherical microparticles having a surface covered with an inorganic oxide having a refractive index of 1.80 or more, and the inorganic oxide is, for example, titanium oxide.

PTL 3 discloses a cosmetic including, as an ultraviolet screening agent, an amorphous phosphate of Ce and/or Ti, and the amorphous phosphate contains, as a crystallization inhibitor, at least one element of B, Al, Si, Zn, Ga, Zr, Nb, Mo, Ta, and W.

### Citation List

### Patent Literature

PTL 1: JP 2007-291090 A
PTL 2: JP 10-167929 A
PTL 3: JP 4649102 B

### Summary of Invention

### Technical Problem

Titanium oxide, however, has a whiteness of less than 100 (relative value when a barium sulfate sample has a whiteness of 100) and has a slightly yellowish color tone. Hence, a white pigment for cosmetics having a higher function as a base pigment than that of titanium oxide is demanded.

The cosmetic disclosed in PTL 2 still has room for improvement in giving a moderate covering function and natural finish without white powdery finish.

A first object of the present invention is to provide a white pigment for cosmetics having a higher function as a base pigment than that of titanium oxide.

A second object of the present invention is to provide a white pigment for cosmetics that gives a moderate covering function and natural finish without white powdery finish when the white pigment is included in a cosmetic composition.

### Solution to Problem

To solve the problems, a white pigment for cosmetics as a first aspect of the present invention includes a titanium phosphate powder having a whiteness of 92.91 or more as determined in accordance with JIS Z 8715.

A white pigment for cosmetics as a second aspect of the present invention includes a titanium phosphate powder having a refractive index of 1.67 or more and 1.83 or less.

### Advantageous Effects of Invention

The white pigment for cosmetics as the first aspect of the present invention has a higher function as a base pigment than that of titanium oxide.

The white pigment for cosmetics as the second aspect of the present invention should give a moderate covering function and natural finish without white powdery finish when included in a cosmetic composition.

### Brief Description of Drawings

Fig. 1 is a scanning electron micrograph of a powder produced in Example 1; and
Fig. 2 is a scanning electron micrograph of a powder produced in Example 3.

### Description of Embodiments

White pigments for cosmetics as first aspect and second aspect

As described above, the white pigment for cosmetics as the first aspect of the present invention includes a titanium phosphate powder having a whiteness of 92.91 or more as determined in accordance with JIS Z 8715. The white pigment for cosmetics as the second aspect includes a titanium phosphate powder having a refractive index of 1.67 or more and 1.83 or less.

The white pigments for cosmetics as the first aspect and the second aspect are a white pigment for cosmetics including a titanium phosphate powder, the titanium phosphate powder includes crystal particles of titanium phosphate, and the ratio of an oil absorption value (ml/100 g) to a specific surface area (m²/g) of the crystal particles (oil absorption value/specific surface area) is 2.0 or more, for example.

The white pigments for cosmetics as the first aspect and the second aspect are a white pigment for cosmetics including a titanium phosphate powder, the titanium phosphate powder includes crystal particles of titanium phosphate, and the titanium phosphate powder has an average friction coefficient (MIU) of less than 1.45, for example.

In the white pigments for cosmetics as the first aspect and the second aspect, the titanium phosphate powder has an average primary particle diameter of 0.05 µm or more and 20 µm or less, for example.

In the white pigments for cosmetics as the first aspect and the second aspect, the titanium phosphate powder includes crystal particles of titanium phosphate, for example.

In the white pigments for cosmetics as the first aspect and the second aspect, the crystal particles are plate crystal particles, for example.

In the white pigments for cosmetics as the first aspect and the second aspect, the plate crystal particles have an average thickness of 0.01 µm or more and 4 µm or less, and an aspect ratio that is a value calculated by dividing an average primary particle diameter of the plate crystal particles by the average thickness is 5 or more, for example.

A composition including the white pigments for cosmetics as the first aspect and the second aspect can be used as a cosmetic.

### Embodiments

Embodiments of the present invention will now be described, but the present invention is not limited to the embodiments described below. The embodiments described below include technically preferred limitations for carrying out the present invention, but the limitations are not essential requirements of the invention.

### First embodiment

A white pigment for cosmetics in a first embodiment includes a titanium phosphate powder having a whiteness of 100.51 as determined in accordance with JIS Z 8715.

The titanium phosphate powder includes plate-shaped titanium phosphate crystal particles. The plate crystals have an average primary particle diameter (for example, a value calculated from, as particle diameters, diameters determined by an image analysis method in which a plate is converted into a circle) of 0.05 µm or more and 20 µm or less, an average thickness of 0.01 µm or more and 4 µm or less, and an aspect ratio (a value calculated by dividing an average primary particle diameter by an average thickness) of 5 or more.

The titanium phosphate powder has a refractive index of 1.79. The titanium phosphate powder has an oil absorption value of 116 ml/100 g as determined in accordance with JIS K 5101-13. The titanium phosphate powder has a ratio of an oil absorption value (ml/100 g) to a specific surface area (m²/g) of the titanium phosphate crystal particles (oil absorption value/specific surface area) of 2.0 or more.

The white pigment for cosmetics in the embodiment has a high whiteness of 100.51 and thus can exhibit a high function as a base pigment that is mixed with other pigments to give a cosmetic composition.

The white pigment for cosmetics in the embodiment has a refractive index of 1.79, which is moderately higher than the refractive index (1.5) of the human skin, and thus a cosmetic composition containing the white pigment can give a cosmetic achieving a moderate covering function and natural finish without white powdery finish.

The white pigment for cosmetics in the embodiment includes a titanium phosphate powder including crystal particles of titanium phosphate and has a ratio of an oil absorption value (ml/100 g) to a specific surface area (m²/g) of the crystal particles (oil absorption value/specific surface area) of 2.0 or more. Hence, a cosmetic containing the white pigment gives a coating film that has less stickiness and is unlikely to undergo makeup deterioration by sebum.

The white pigment for cosmetics in the embodiment includes a titanium phosphate powder including plate crystal particles of titanium phosphate. The titanium phosphate powder has an average primary particle diameter of 0.05 µm or more and 20 µm or less, and the plate crystal particles have an average thickness of 0.01 µm or more and 4 µm or less and an aspect ratio of 5 or more. The titanium phosphate powder has an average friction coefficient (MIU) of less than 1.45. Accordingly, a cosmetic containing the white pigment has excellent slipperiness. In other words, a cosmetic containing the white pigment for cosmetics in the embodiment can be smoothly applied onto the skin.

The titanium phosphate powder can be produced by the following method, for example.

First, an aqueous solution of titanium sulfate and an aqueous solution of phosphoric acid are mixed at such a ratio that the phosphorus molarity [P] to the titanium molarity [Ti], [P] / [Ti], is 5 or more and 21 or less, giving a liquid mixture. Next, the liquid mixture is placed in a closed container and is maintained at a temperature of 100°C or more and 160°C or less to undergo reaction for a predetermined period (for example, 5 hours or more). In other words, hydrothermal synthesis is performed. The pressure in the closed container is higher than the atmospheric pressure and is naturally determined by a pressing temperature. A slurry containing crystal particles of titanium phosphate is thus prepared.

Next, the prepared slurry is cooled, and then a solid content (crystal particles of titanium phosphate) is separated from the slurry. The resulting solid content is cleaned with a cleaning solution containing aqueous ammonia (ammonium hydroxide) and then is dried.

### Second embodiment

A white pigment for cosmetics in a second embodiment includes a titanium phosphate powder having a whiteness of 97.15 as determined in accordance with JIS Z 8715.

The titanium phosphate powder includes plate-shaped titanium phosphate crystal particles. The plate crystals have an average primary particle diameter (for example, a value calculated from, as particle diameters, diameters determined by an image analysis method in which a plate is converted into a circle) of 0.05 µm or more and 20 µm or less, an average thickness of 0.01 µm or more and 4 µm or less, and an aspect ratio (a value calculated by dividing an average primary particle diameter by an average thickness) of 5 or more.

The titanium phosphate powder has a refractive index of 1.79. The titanium phosphate powder has an oil absorption value of 116 ml/100 g as determined in accordance with JIS K 5101-13.

The titanium phosphate powder can be produced by the following method, for example.

First, the same procedure as in the first embodiment is performed to give a slurry containing crystal particles of titanium phosphate. The slurry is cooled, and then a solid content (crystal particles of titanium phosphate) is separated from the slurry. The resulting solid content is cleaned with water and then is dried.

### Third embodiment

A white pigment for cosmetics in a third embodiment includes a titanium phosphate powder having a whiteness of 96.32 to 97.47 as determined in accordance with JIS Z 8715.

The titanium phosphate powder includes plate-shaped titanium phosphate crystal particles. The plate crystals have an average primary particle diameter (for example, a value calculated from, as particle diameters, diameters determined by an image analysis method in which a plate is converted into a circle) of 0.05 µm or more and 20 µm or less, an average thickness of 0.01 µm or more and 2 µm or less, and an aspect ratio (a value calculated by dividing an average primary particle diameter by an average thickness) of 5 or more.

The titanium phosphate powder has a refractive index of 1.73 to 1.83. The titanium phosphate powder has an oil absorption value of 45 ml/100 g or more and 77 ml/100 g or less as determined in accordance with JIS K 5101-13. The titanium phosphate powder has a ratio of an oil absorption value (ml/100 g) to a specific surface area (m²/g) of the titanium phosphate crystal particles (oil absorption value/specific surface area) of 2.0 or more.

The white pigment for cosmetics in the embodiment has a high whiteness of 96.32 to 97.47 and thus can exhibit a high function as a base pigment that is mixed with other pigments to give a cosmetic composition.

The white pigment for cosmetics in the embodiment has a refractive index of 1.73 to 1.83, which is moderately higher than the refractive index (1.5) of the human skin, and thus a cosmetic composition containing the white pigment can give a cosmetic achieving a moderate covering function and natural finish without white powdery finish.

The white pigment for cosmetics in the embodiment includes a titanium phosphate powder including crystal particles of titanium phosphate and has a ratio of an oil absorption value (ml/100 g) to a specific surface area (m²/g) of the crystal particles (oil absorption value/specific surface area) of 2.0 or more. Hence, a cosmetic containing the white pigment gives a coating film that has less stickiness and is unlikely to undergo makeup deterioration by sebum.

The white pigment for cosmetics in the embodiment includes a titanium phosphate powder including plate crystal particles of titanium phosphate. The titanium phosphate powder has an average primary particle diameter of 0.05 µm or more and 20 µm or less, and the plate crystal particles have an average thickness of 0.01 µm or more and 4 µm or less and an aspect ratio of 5 or more. The titanium phosphate powder has an average friction coefficient (MIU) of less than 1.45. Accordingly, a cosmetic containing the white pigment has excellent slipperiness. In other words, a cosmetic containing the white pigment for cosmetics in the embodiment can be smoothly applied onto the skin.

The titanium phosphate powder can be produced by the following method, for example.

First, an aqueous solution of titanyl sulfate and an aqueous solution of phosphoric acid are mixed at such a ratio that the phosphorus molarity [P] to the titanium molarity [Ti], [P] / [Ti], is 5 or more and 21 or less, giving a liquid mixture. Next, the liquid mixture is placed in a closed container and is maintained at a temperature of 100°C or more and 160°C or less to undergo reaction for a predetermined period (for example, 5 hours or more). In other words, hydrothermal synthesis is performed. The pressure in the closed container is higher than the atmospheric pressure and is naturally determined by a pressing temperature. A slurry containing crystal particles of titanium phosphate is thus prepared.

Next, the prepared slurry is cooled, and then a solid content (crystal particles of titanium phosphate) is separated from the slurry. The resulting solid content is cleaned with water and then is dried.

### Fourth embodiment

A white pigment for cosmetics in a fourth embodiment includes a titanium phosphate powder having a whiteness of 92.91 as determined in accordance with JIS Z 8715.

The titanium phosphate powder includes an amorphous titanium phosphate and has an average primary particle diameter (for example, a value calculated from, as particle diameters, diameters determined by an image analysis method in which a particle is converted into a sphere) of 0.05 µm. The titanium phosphate powder has a refractive index of 1.67.

The titanium phosphate powder can be produced by the following method, for example.

First, an aqueous solution of titanium sulfate and an aqueous solution of phosphoric acid are placed in an open container at such a ratio that the phosphorus molarity [P] to the titanium molarity [Ti], [P]/[Ti], is 1.33, and the whole is mixed without heat. In other words, room temperature synthesis is performed. This reaction generates an amorphous titanium phosphate. The amorphous titanium phosphate is cleaned, then dried, and pulverized.

In the above embodiments, titanium sulfate and titanyl sulfate are used as the titanium source to give titanium phosphate powders as examples, and other examples of the titanium source include peroxotitanic acid.

The titanium phosphate powder included in the white pigment for cosmetics preferably includes plate crystal particles of titanium phosphate having an average primary particle diameter of 0.1 µm or more and 20 µm or less and an aspect ratio of 5 or more. The average primary particle diameter is more preferably 0.2 µm or more and 10 µm or less, and the aspect ratio is more preferably 9 or more. The crystal particles included in the titanium phosphate powder included in the white pigment for cosmetics preferably have a ratio of an oil absorption value (ml/100 g) to a specific surface area (m²/g) (oil absorption value/specific surface area) of 3.0 or more.

A cosmetic containing a powder having a higher ratio (oil absorption value/specific surface area) gives a coating film that has less stickiness and is unlikely to undergo makeup deterioration by sebum, but a powder having an excessively high ratio unfortunately gives compounds having larger variations in viscosity, bulk density, and the like when a cosmetic is produced. From this viewpoint, the ratio is preferably 200.0 or less.

The average friction coefficient (MIU) is preferably 1.40 or less and more preferably 1.35 or less. A cosmetic containing a powder having a smaller average friction coefficient (MIU) can be more smoothly applied onto the skin, but a cosmetic containing a powder having an excessively small average friction coefficient has poor adhesion to the skin. From this viewpoint, the average friction coefficient is preferably 0.20 or more and more preferably 0.65 or more.

### With regard to cosmetic

Examples of the cosmetic including a composition containing a white pigment including a powder (hereinafter called "cosmetic composition") include makeup cosmetics such as a foundation, a face powder, a cheek rouge, and an eye shadow; and skin care cosmetics such as a whitening powder and a body powder. The white pigments in the first embodiment to the fifth embodiment are suitable for a white pigment in these cosmetic compositions.

When the white pigment is used as an additive in a foundation or the like, which is required to have a covering function, brightness, or the like, a titanium phosphate powder having an average primary particle diameter of 1 µm or more and 20 µm or less is preferably used.

### Examples

### Example 1

First, an aqueous solution of titanium sulfate and an aqueous solution of phosphoric acid were mixed at such a ratio that the phosphorus molarity [P] to the titanium molarity [Ti], [P] / [Ti], was 13, giving a liquid mixture. Next, the liquid mixture was placed in a closed container (an internal volume of 100 mL) and was maintained at a temperature of 110°C to undergo reaction for 5 hours.

After the reaction, the lid was removed, a slurry in the container was cooled to room temperature and then was taken out of the container, and a solid content was separated from the slurry by filtration. The solid content was cleaned with 29% aqueous ammonia (an aqueous solution of an ammonium salt) and then was dried (by standing at a temperature of 105°C for 24 hours), giving a powder.

The resulting powder was analyzed by using an X-ray diffractometer, and the result revealed that the particles included in the powder were a crystalline titanium phosphate having a structural formula of Ti(HPO₄)₂·H₂O.

The whiteness of the resulting powder was determined by using an ultraviolet and visible spectrophotometer "UV-2450" manufactured by Shimadzu Corporation with an illuminant D65 in a condition of a visual field of 2° to be 100.51. In other words, the resulting powder had a whiteness of 100.51 as determined in accordance with JIS Z 8715.

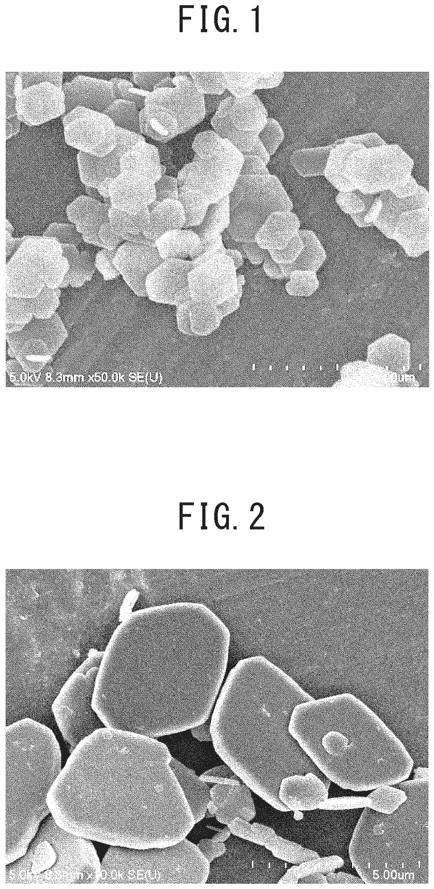

The resulting powder was observed under a scanning electron microscope, and the result revealed that, as illustrated in Fig. 1, the particles included in the powder had a plate-like shape, and many hexagonal plates were included. From an image under the scanning electron microscope, the average thickness of the crystal particles included in the resulting powder was determined to be 0.017 µm. An image under the scanning electron microscope was analyzed by using an image analysis software "Mac-View ver. 4" manufactured by Mountech Co. Ltd., and the average primary particle diameter of the crystal particles included in the resulting powder was determined to be 0.24 µm. Calculation was performed by using these measured values (0.24/0.017), and the aspect ratio of the crystal particles included in the resulting powder was determined to be 14.

The refractive index of the resulting powder was determined by the following method to be 1.79.

First, the resulting powder and polymethyl methacrylate (film substrate: a transparent resin to be a film base) were added to and mixed with N-methylpyrrolidone (solvent capable of dissolving the film substrate) to give a liquid in which the powder was dispersed and polymethyl methacrylate was dissolved. The content of the powder was changed to give a plurality of liquids. Such a liquid was used to form a coated film having a thickness of 600 µm on a PET film, and then the coated film was dried at 80°C to give a film including only the powder and the resin. After cooling, the film was released from the PET film.

Each refractive index of a plurality of the films prepared as above was determined by using a refractometer "Prism Coupler, model 2010/M" manufactured by Metricon with a helium-neon laser beam having a wavelength of 632.8 nm as a light source . The measured values of the refractive indexes of the plurality of films were plotted on a graph with powder content (% by volume) as the horizontal axis and refractive index as the vertical axis, and the plots were approximated by a straight line. The straight line was extrapolated to a point at which the powder content was 100%, and the refractive index at the point was regarded as the refractive index of the powder.

The oil absorption value of 100 g of the resulting powder was determined by a method in accordance with JIS K 5101-13 to be 116 ml/100 g. The specific surface area of the resulting powder was determined by using a fully automatic specific surface area analyzer "Macsorb (registered trademark) HM-1210" manufactured by Mountech Co. Ltd. by BET fluid process to be 25.0 m²/g. Calculation was performed by using these measured values (116/25.0), and the ratio of an oil absorption value (ml/100 g) to a specific surface area (m²/g) of the crystal particles included in the resulting powder (oil absorption value/specific surface area) was determined to be 4.6.

The average friction coefficient (MIU) of the resulting powder was determined by using a friction tester "KES-SE" manufactured by Kato Tech to be 1.37. The measurement was performed by using a 10-mm square silicon sensor in conditions of a static load of 25 g and a scan speed of 1 mm/sec.

The resulting powder had a bulk density of 0.13 g/ml and a specific gravity of 2.59. The resulting powder had no photocatalytic activity.

A cosmetic composition containing the resulting powder as a white pigment was prepared. The resulting powder had a high whiteness (100.51) and thus was able to exhibit a high function as the base pigment. In addition, the resulting powder had a moderately higher refractive index (1.79) than the refractive index (1.5) of the human skin, and thus use of the prepared cosmetic composition achieved a moderate covering function and natural finish without white powdery finish.

Moreover, the resulting powder had an "oil absorption value/specific surface area" of 4.6 (in the range not less than 2.0), and thus the prepared cosmetic composition gave a coating film having less stickiness and lasting long. The resulting powder had a plate-like crystal shape (an aspect ratio of 14) and had an average friction coefficient (MIU) of 1.37, and thus the prepared cosmetic composition also had excellent slipperiness. In other words, the cosmetic containing the powder was able to be smoothly applied onto the skin.

### Example 2

First, an aqueous solution of titanium sulfate and an aqueous solution of phosphoric acid were mixed at such a ratio that the phosphorus molarity [P] to the titanium molarity [Ti], [P] / [Ti], was 21, giving a liquid mixture. Next, the liquid mixture was placed in a closed container (an internal volume of 100 mL) and was maintained at a temperature of 160°C to undergo reaction for 5 hours.

After the reaction, the lid was removed, a slurry in the container was cooled to room temperature and then was taken out of the container, and a solid content was separated from the slurry by filtration. The solid content was cleaned with water and then was dried (by standing at a temperature of 105°C for 24 hours), giving a powder.

The resulting powder was analyzed by using an X-ray diffractometer, and the result revealed that the particles included in the powder were a crystalline titanium phosphate having a structural formula of Ti(HPO₄)₂·H₂O.

The whiteness of the resulting powder was determined by using an ultraviolet and visible spectrophotometer "UV-2450" manufactured by Shimadzu Corporation with an illuminant D65 in a condition of a visual field of 2° to be 97.75. In other words, the resulting powder had a whiteness of 97.75 as determined in accordance with JIS Z 8715.

The resulting powder was observed under a scanning electron microscope, and the result revealed that, as with Example 1, the particles included in the powder had a plate-like shape, and many hexagonal plates were included. From an image under the scanning electron microscope, the average thickness of the crystal particles included in the resulting powder was determined to be 0.026 µm. An image under the scanning electron microscope was analyzed by using an image analysis software "Mac-View ver. 4" manufactured by Mountech Co. Ltd., and the average primary particle diameter of the crystal particles included in the resulting powder was determined to be 0.24 µm. Calculation was performed by using these measured values (0.24/0.026), and the aspect ratio of the crystal particles included in the resulting powder was determined to be 9.

The refractive index of the resulting powder was determined by the same method as in Example 1 to be 1.79. In other words, the resulting powder had a refractive index of 1.79 as determined in accordance with JIS K 7142.

The oil absorption value of 100 g of the resulting powder was determined by a method in accordance with JIS K 5101-13 to be 116 ml/100 g.

The resulting powder had a bulk density of 0.13 g/ml and a specific gravity of 2.59. The resulting powder had no photocatalytic activity.

A cosmetic composition containing the resulting powder as a white pigment was prepared. The resulting powder had a high whiteness (97.75) and thus was able to exhibit a high function as the base pigment. In addition, the resulting powder had a moderately higher refractive index (1.79) than the refractive index (1.5) of the human skin, and thus use of the prepared cosmetic composition achieved a moderate covering function and natural finish without white powdery finish. Moreover, the resulting powder had a high oil absorption value, and thus the prepared cosmetic composition had long-lasting properties. The resulting powder had a plate-like crystal shape, and thus the prepared cosmetic composition also had excellent slipperiness.

### Example 3

First, an aqueous solution of titanyl sulfate and an aqueous solution of phosphoric acid were mixed at such a ratio that the phosphorus molarity [P] to the titanium molarity [Ti], [P] / [Ti], was 11, giving a liquid mixture. Next, the liquid mixture was placed in a closed container (an internal volume of 100 mL) and was maintained at a temperature of 130°C to undergo reaction for 5 hours.

After the reaction, the lid was removed, a slurry in the container was cooled to room temperature and then was taken out of the container, and a solid content was separated from the slurry by filtration. The solid content was cleaned with water and then was dried (by standing at a temperature of 105°C for 24 hours), giving a powder.

The resulting powder was analyzed by using an X-ray diffractometer, and the result revealed that the particles included in the powder were a crystalline titanium phosphate having a structural formula of Ti(HPO₄)₂·H₂O.

The whiteness of the resulting powder was determined by using an ultraviolet and visible spectrophotometer "UV-2450" manufactured by Shimadzu Corporation with an illuminant D65 in a condition of a visual field of 2° to be 96.32. In other words, the resulting powder had a whiteness of 96.32 as determined in accordance with JIS Z 8715.

The resulting powder was observed under a scanning electron microscope, and the result revealed that, as illustrated in Fig. 2, the particles included in the powder had a plate-like shape, and many hexagonal plates were included. From an image under the scanning electron microscope, the average thickness of the crystal particles included in the resulting powder was determined to be 0.27 µm. An image under the scanning electron microscope was analyzed by using an image analysis software "Mac-View ver. 4" manufactured by Mountech Co. Ltd., and the average primary particle diameter of the crystal particles included in the resulting powder was determined to be 3.04 µm. Calculation was performed by using these measured values (3.04/0.27), and the aspect ratio of the crystal particles included in the resulting powder was determined to be 11.

The refractive index of the resulting powder was determined by the same method as in Example 1 to be 1.73.

The oil absorption value of 100 g of the resulting powder was determined by a method in accordance with JIS K 5101-13 to be 45 ml/100 g. The specific surface area of the resulting powder was determined by using a fully automatic specific surface area analyzer "Macsorb (registered trademark) HM-1210" manufactured by Mountech Co. Ltd. by BET fluid process to be 1.65 m²/g. Calculation was performed by using these measured values (45/1.65), and the ratio of an oil absorption value (ml/100 g) to a specific surface area (m²/g) of the crystal particles included in the resulting powder (oil absorption value/specific surface area) was determined to be 27.3.

The average friction coefficient (MIU) of the resulting powder was determined by using a friction tester "KES-SE" manufactured by Kato Tech to be 0.99. The measurement was performed by using a 10-mm square silicon sensor in conditions of a static load of 25 g and a scan speed of 1 mm/sec.

The resulting powder had a bulk density of 0.13 g/ml and a specific gravity of 2.59. The resulting powder had no photocatalytic activity.

A cosmetic composition containing the resulting powder as a white pigment was prepared. The resulting powder had a high whiteness (96.32) and thus was able to exhibit a high function as the base pigment. In addition, the resulting powder had a moderately higher refractive index (1.73) than the refractive index (1.5) of the human skin, and thus use of the prepared cosmetic composition achieved a moderate covering function and natural finish without white powdery finish. Moreover, the resulting powder had an "oil absorption value/specific surface area" of 27.3 (in the range not less than 2.0), and thus the prepared cosmetic composition gave a coating film having less stickiness and lasting long.

The resulting powder had a plate-like crystal shape (an aspect ratio of 11) and had an average friction coefficient (MIU) of 0.99, and thus the prepared cosmetic composition also had excellent slipperiness. In other words, the cosmetic containing the powder was able to be smoothly applied onto the skin.

### Example 4

First, an aqueous solution of titanyl sulfate and an aqueous solution of phosphoric acid were mixed at such a ratio that the phosphorus molarity [P] to the titanium molarity [Ti], [P] / [Ti], was 13, giving a liquid mixture. Next, the liquid mixture was placed in a closed container (an internal volume of 100 mL) and was maintained at a temperature of 110°C to undergo reaction for 5 hours.

After the reaction, the lid was removed, a slurry in the container was cooled to room temperature and then was taken out of the container, and a solid content was separated from the slurry by filtration. The solid content was cleaned with water and then was dried (by standing at a temperature of 105°C for 24 hours), giving a powder.

The resulting powder was analyzed by using an X-ray diffractometer, and the result revealed that the particles included in the powder were a crystalline titanium phosphate having a structural formula of Ti(HPO₄)₂·H₂O.

The whiteness of the resulting powder was determined by using an ultraviolet and visible spectrophotometer "UV-2450" manufactured by Shimadzu Corporation with an illuminant D65 in a condition of a visual field of 2° to be 97.47. In other words, the resulting powder had a whiteness of 97.47 as determined in accordance with JIS Z 8715.

The resulting powder was observed under a scanning electron microscope, and the result revealed that, as with Example 1, the particles included in the powder had a plate-like shape, and many hexagonal plates were included. From an image under the scanning electron microscope, the average thickness of the crystal particles included in the resulting powder was determined to be 0.026 µm. An image under the scanning electron microscope was analyzed by using an image analysis software "Mac-View ver. 4" manufactured by Mountech Co. Ltd., and the average primary particle diameter of the crystal particles included in the resulting powder was determined to be 0.30 µm. Calculation was performed by using these measured values (0.30/0.026), and the aspect ratio of the crystal particles included in the resulting powder was determined to be 12.

The refractive index of the resulting powder was determined by the same method as in Example 1 to be 1.83. In other words, the resulting powder had a refractive index of 1.83 as determined in accordance with JIS K 7142.

The oil absorption value of 100 g of the resulting powder was determined by a method in accordance with JIS K 5101-13 to be 77 ml/100 g. The specific surface area of the resulting powder was determined by using a fully automatic specific surface area analyzer "Macsorb (registered trademark) HM-1210" manufactured by Mountech Co. Ltd. by BET fluid process to be 22.7 m²/g. Calculation was performed by using these measured values (77/22.7), and the ratio of an oil absorption value (ml/100 g) to a specific surface area (m²/g) of the crystal particles included in the resulting powder (oil absorption value/specific surface area) was determined to be 3.4.

The resulting powder had a bulk density of 0.13 g/ml and a specific gravity of 2.59. The resulting powder had no photocatalytic activity.

A cosmetic composition containing the resulting powder as a white pigment was prepared. The resulting powder had a high whiteness (97.47) and thus was able to exhibit a high function as the base pigment. In addition, the resulting powder had a moderately higher refractive index (1.83) than the refractive index (1.5) of the human skin, and thus use of the prepared cosmetic composition achieved a moderate covering function and natural finish without white powdery finish. Moreover, the resulting powder had an "oil absorption value/specific surface area" of 3.4 (in the range not less than 2.0), and thus the prepared cosmetic composition gave a coating film having less stickiness and lasting long. The resulting powder had a plate-like crystal shape (an aspect ratio of 12), and thus the prepared cosmetic composition also had excellent slipperiness.

### Example 5

First, an aqueous solution of titanium sulfate and an aqueous solution of phosphoric acid were placed in an open vessel at such a ratio that the phosphorus molarity [P] to the titanium molarity [Ti], [P] / [Ti], was 1.3, and the whole was stirred without heat. As a result, an amorphous gel was formed, and the open vessel contained a mixture of the gel and water.

Next, the mixture was cleaned with water and was filtered to collect the gel. The gel was dried (by standing at a temperature of 105°C for 24 hours), and the dried product was crushed by using a jet mill, giving a powder.

The resulting powder was analyzed by using an X-ray diffractometer and a fluorescent X-ray analyzer, and the result revealed that the particles included in the powder were an amorphous titanium phosphate having a structural formula of Ti₃(HPO₄)₄.

The whiteness of the resulting powder was determined by using an ultraviolet and visible spectrophotometer "UV-2450" manufactured by Shimadzu Corporation with an illuminant D65 in a condition of a visual field of 2° to be 92.91. In other words, the resulting powder had a whiteness of 92.91 as determined in accordance with JIS Z 8715.

The resulting powder was observed under a scanning electron microscope. An image under the scanning electron microscope was analyzed by using an image analysis software "Mac-View ver. 4" manufactured by Mountech Co. Ltd., and the average primary particle diameter of the resulting powder was determined to be 0.05 µm.

The refractive index of the resulting powder was determined by the same method as in Example 1 to be 1.67.

The oil absorption value of 100 g of the resulting powder was determined by a method in accordance with JIS K 5101-13 to be 72 ml/100 g. The specific surface area of the resulting powder was determined by using a fully automatic specific surface area analyzer "Macsorb (registered trademark) HM-1210" manufactured by Mountech Co. Ltd. by BET fluid process to be 62.6 m²/g. Calculation was performed by using these measured values (72/62.6), and the ratio of an oil absorption value (ml/100 g) to a specific surface area (m²/g) of the crystal particles included in the resulting powder (oil absorption value/specific surface area) was determined to be 1.2.

The resulting powder had no photocatalytic activity.

A cosmetic composition containing the resulting powder as a white pigment was prepared. The resulting powder had a high whiteness (92.91) and thus was able to exhibit a high function as the base pigment. In addition, the resulting powder had a moderately higher refractive index (1.67) than the refractive index (1.5) of the human skin, and thus use of the prepared cosmetic composition achieved a moderate covering function and natural finish without white powdery finish.

However, the resulting powder had an "oil absorption value/specific surface area" of 1.2 (out of the range not less than 2.0), and thus the cosmetic composition prepared in Example 5 gave a coating film having high stickiness and was likely to cause makeup deterioration as compared with the cosmetic compositions prepared in Examples 1 to 4. The resulting powder included amorphous titanium phosphate particles, and thus the cosmetic composition prepared in Example 5 was inferior in slipperiness to the cosmetic compositions prepared in Examples 1 to 4.

### Comparative Example 1

As a rutile-type titanium oxide powder, "titanium(IV) oxide, rutile-type" was purchased from Kanto Chemical Co., Inc. The whiteness of the titanium oxide powder was determined by using an ultraviolet and visible spectrophotometer "UV-2450" manufactured by Shimadzu Corporation with an illuminant D65 in a condition of a visual field of 2° to be 91.89. In other words, the titanium oxide powder had a whiteness of 91.89 as determined in accordance with JIS Z 8715.

The refractive index of the titanium oxide powder was determined by the same method as in Example 1 to be 2.6.

The oil absorption value of 100 g of the titanium oxide powder was determined by a method in accordance with JIS K 5101-13 to be 18 ml/100 g.

An image of the titanium oxide powder under a scanning electron microscope was analyzed by using an image analysis software "Mac-View ver. 4" manufactured by Mountech Co. Ltd., and the average primary particle diameter of the titanium oxide powder was determined to be 0.05 µm.

A cosmetic composition containing the titanium oxide powder as a white pigment was prepared. The titanium oxide powder had a low whiteness (91.89) and thus exhibited an insufficient function as the base pigment. In addition, the titanium oxide powder had an excessively higher refractive index (2.6) than the refractive index (1.5) of the human skin, and thus the prepared cosmetic composition had a covering function but was likely to give white powdery finish. The titanium oxide powder had a low oil absorption value, and thus the prepared cosmetic composition was likely to cause makeup deterioration by sebum.

### Comparative Example 2

As a rutile-type titanium oxide powder, particulate titanium oxide "MT-500B" was purchased from Tayca Corporation. The titanium oxide powder had an average primary particle diameter of 35 nm (according to a catalog). The whiteness of the titanium oxide powder was determined by using an ultraviolet and visible spectrophotometer "UV-2450" manufactured by Shimadzu Corporation with an illuminant D65 in a condition of a visual field of 2° to be 92.47. In other words, the titanium oxide powder had a whiteness of 92.47 as determined in accordance with JIS Z 8715.

The refractive index of the titanium oxide powder was determined by the same method as in Example 1 to be 2.6.

The oil absorption value of 100 g of the titanium oxide powder was determined by a method in accordance with JIS K 5101-13 to be 62 ml/100 g. The specific surface area of the resulting powder was determined by using a fully automatic specific surface area analyzer "Macsorb (registered trademark) HM-1210" manufactured by Mountech Co. Ltd. by BET fluid process to be 40.4 m²/g. Calculation was performed by using these measured values (62/40.4), and the ratio of an oil absorption value (ml/100 g) to a specific surface area (m²/g) of the crystal particles included in the resulting powder (oil absorption value/specific surface area) was determined to be 1.5.

The average friction coefficient (MIU) of the resulting powder was determined by using a friction tester "KES-SE" manufactured by Kato Tech to be 1.52. The measurement was performed by using a 10-mm square silicon sensor in conditions of a static load of 25 g and a scan speed of 1 mm/sec.

A cosmetic composition containing the titanium oxide powder as a white pigment was prepared. The titanium oxide powder had a low whiteness (92.47) and thus exhibited an insufficient function as the base pigment. In addition, the titanium oxide powder had an excessively higher refractive index (2.6) than the refractive index (1.5) of the human skin, and thus the prepared cosmetic composition had a covering function but was likely to cause white powdery finish. Moreover, the resulting powder had an "oil absorption value/specific surface area" of 1.5 (out of the range not less than 2.0), and thus the prepared cosmetic composition gave a coating film having high stickiness and was likely to cause makeup deterioration by sebum.

The titanium oxide powder had no plate-like crystal shape and had an average friction coefficient (MIU) of 1.52, and thus the cosmetic composition prepared in Comparative Example 2 was inferior in slipperiness to the cosmetic compositions prepared in Examples 1 and 3. In other words, the cosmetic containing the powder failed to be smoothly applied onto the skin.

Physical properties and the like in these examples and comparative examples are listed in Table 1.

**[Table 1]**

| | Material of powder | Ti material | P material | P/Ti | Synthetic conditions | Cleaning solution | Whiteness | Average thickness (µm) | Average primary particle diameter (µm) | Aspect ratio | Refractive index | Oil absorption value (ml/100g) | SA (m²/g) | Oil absorption value/SA | MIU |
|---|---|---|---|---|---|---|---|---|---|---|---|---|---|---|---|
| Example 1 | Crystalline titanium phosphate | Titanium sulfate | Phosphoric acid | 13 | 110°C, 5H | Aqueous ammonia | 100.5 | 0.017 | 0.24 | 14 | 1.79 | 116 | 25.0 | 4.6 | 1.37 |
| Example 2 | Crystalline titanium phosphate | Titanium sulfate | Phosphoric acid | 21 | 160°C, 5H | Water | 97.75 | 0.026 | 0.24 | 9 | 1.79 | 116 | - | - | - |
| Example 3 | Crystalline titanium phosphate | Titanyl sulfate | Phosphoric acid | 11 | 130°C, 5H | Water | 96.32 | 0.27 | 3.04 | 11 | 1.73 | 45 | 1.65 | 27.3 | 0.99 |
| Example 4 | Crystalline titanium phosphate | Titanyl sulfate | Phosphoric acid | 13 | 110°C, 5H | Water | 97.47 | 0.026 | 0.30 | 12 | 1.83 | 77 | 22.7 | 3.4 | - |
| Example 5 | Amorphous titanium phosphate | Titanium sulfate | Phosphoric acid | 1.3 | Room temperature | Water | 92.91 | - | 0.05 | - | 1.67 | 72 | 62.6 | 1.2 | - |
| Comparative Example 1 | Rutile-type titanium oxide | - | - | - | - | - | 91.89 | - | 0.05 | - | 2.60 | 18 | - | - | - |
| Comparative Example 2 | Rutile-type titanium oxide | - | - | - | - | - | 92.47 | - | 0.035 | - | 2.60 | 62 | 40.4 | 1.5 | 1.52 |

The above results reveal that the white pigment for cosmetics including the titanium phosphate powder has a higher function as a base material than that of titanium oxide.

## Claims

1. A white pigment for cosmetics, the white pigment comprising:
a titanium phosphate powder having a whiteness of 92.91 or more as determined in accordance with JIS Z 8715.

2. A white pigment for cosmetics, the white pigment comprising:
a titanium phosphate powder having a refractive index of 1.67 or more and 1.83 or less.

3. The white pigment for cosmetics according to claim 1 or 2, wherein the titanium phosphate powder has an average primary particle diameter of 0.05 µm or more and 20 µm or less.

4. The white pigment for cosmetics according to any one of claims 1 to 3, wherein the titanium phosphate powder includes crystal particles of titanium phosphate.

5. The white pigment for cosmetics according to claim 4, wherein the crystal particles are plate crystal particles.

6. The white pigment for cosmetics according to claim 5, wherein
the plate crystal particles have an average thickness of 0.01 µm or more and 4 µm or less, and
an aspect ratio that is a value calculated by dividing an average primary particle diameter of the plate crystal particles by the average thickness is 5 or more.

7. A cosmetic comprising:
a composition containing the white pigment for cosmetics according to claim 1.

8. A cosmetic comprising:
a composition containing the white pigment for cosmetics according to claim 2.
